# EUROPEAN PATENT APPLICATION

(11) **EP 4 686 417 A1**
(43) Date of publication of application: **04.02.2026**
(21) Application number: 25192644.0
(22) Date of filing: 29.07.2025
(51) Int. Cl.: A23L 27/00, A23P 10/30, A61K 9/107, A61K 9/16, A61K 31/732, B01J 13/04

(54) **NANO/MICROCAPSULES WITH HEALTH-PROMOTING CONTENT, A HEALTH-PROMOTING PRODUCT CONTAINING THESE NANO/MICROCAPSULES AND METHOD FOR OBTAINING THEM**

(30) Priority: 31.07.2024 PL 44942124
(71) Applicant: Uniwersytet Rolniczy im. Hugona Kollataja w Krakowie, 31-120 Krakow (PL)
(72) Inventor: WOSZCZAK, Liliana, 31-467 Krakow (PL); KHACHATRYAN, Karen, 32-083 Balice (PL); KHACHATRYAN, Gohar, 32-083 Balice (PL)
(74) Representative: Godlewski, Piotr

(57) **Abstract**

The subject of the invention are nano/microcapsules with an average diameter of 0.1 µm to 5 µm having a coating with a thickness of 5 nm to 100 nm encapsulating a core, characterised in that the coating (4) of the nano/microcapsules (1) contains pectin supplied for the preparation of the nano/microcapsules (1) in the fruit pulp, and the core (3) is a water-oil nanoemulsion containing bioactive components (6, 7). The subject of the application is also a health-promoting product containing these nano/microcapsules and the method of obtaining them.

## Description

The subject matter of the invention is nano/microcapsules with health-promoting contents obtained from fruit pulp and a health-promoting product, especially in the form of an edible film, pastilles or mousse containing these nano/microcapsules. The subject matter of the invention is also the method of obtaining nano/microcapsules. The product containing nano/microcapsules belongs to the group of food products known as functional foods and/or to the group of food supplements.

Fruit pastilles, formed from edible films, are a natural product prepared from fruit pulp. The advantage of these well-known products is their ease of manufacture. They are obtained by drying the fruit pulp at low temperatures. They contain a lot of vitamins and beneficial trace elements, health-promoting substances, i.e. antioxidants, anthocyanins, which are naturally contained in the fruit. Due to the natural taste of the fruit, they can be enjoyed by consumers of all ages. Depending on the fruit used, the taste can be adjusted without the addition of sugar, acidity regulators or synthetic colours. They are already well-known in Poland and available for sale. These include, for example, fruit wraps: https://www.bob-snail.com/pl/menu/owocowe-zawijasy/ or fruit rolls: https://naturalniezdrowe.pl/przekaski-i-slodycze/792-zestaw-rollsy-witaminowe-5-smakow.html

Fruit is a rich source of antioxidants such as polyphenols (anthocyanins, procyanidins, phenolic acids, flavonols and flavanols), but it also has other essential substances with health-promoting potential [1-5]. Most fruits also contain pectin in their composition. Pectins are a building block of plant cell walls, and all fruits contain them in varying amounts. Blackcurrants, apples, plums and gooseberries are the richest in pectin.

Pectins are water-soluble heteropolysaccharides extracted mainly from fruit. Due to their gelling properties, biocompatibility, and non-toxicity, they can be easily used to make pastilles (edible films) [6].

The above-mentioned properties of pectins also enable their use in the production of nano/microcapsules. Capsules/micelles made from pectin are known in the state of the art, wherein pure pectin, preferably obtained from fruit or produced by other means, is used for their preparation.

In recent years, nanotechnology has been applied to the production of functional foods. One of the directions in functional food production is enriching and increasing the amount of health-promoting and bioactive ingredients in traditional foods. Bioactive compounds are natural antioxidants that have numerous health benefits and anti-disease effects in the prevention and/or treatment of urinary tract infections, cardiovascular diseases, coronary heart disease, metabolic and degenerative diseases, stomach ulcers, and several forms of cancer [7-10]. Despite having many advantages, bioactive compounds are chemically unstable and prone to oxidative degradation. The use of biologically active compounds is also very limited in food and medicinal products due to their rapid release, poor solubility, and low bioavailability. Nano/micro-encapsulation can protect bioactive compounds from environmental stress, improve physicochemical properties and enhance their health-promoting and anti-disease effects and mask undesirable aftertaste [11-14].

It is known from the state of the art that the introduction of biologically active compounds into micro- and/or nanocapsules allows new products with improved physicochemical properties and biological activity to be developed. Depending on the properties of the coating, the resulting products can improve factors such as solubility, absorption and release rate and site, and duration of effect.

There are known methods of encapsulating substances in order to extend shelf life, protect the active substance from loss due to evaporation, stabilize unstable food components against decomposition under the influence of light, oxygen, moisture and other factors, protect against microbial contamination, masking undesirable taste and aroma, improving texture, preventing undesirable interactions between food ingredients, facilitating the use of liquid substances by converting them into solids, enabling controlled gradual release of the active substance, and improving the taste, colour, and appearance of products. The capsule coatings are made primarily from starch, modified starch, sucrose, arabic gum, alginates, carrageenan, cyclodextrins, pectins, gelatin, casein, soy protein, or carnauba wax.

The WO2021082382A1 application discloses microcapsules with one or more layers of a depositing structure, containing: a core and, optionally, at least one coating covering the pellet core. The outer diameter of the pellet core of the microcapsule is about 50 to 500 µm (preferably about 50 to about 300 µm), and the outer diameter of the monolayer or multilayer coated microcapsules is about 200 to about 1000 µm (preferably about 100 to about 500 µm). The deposited substance comprises a functional active substance selected from the group comprising functional polysaccharides, functional lipids, functional proteins/peptides/amino acids, one or more microorganic regulators, vitamins, and minerals. Among the coating materials, pectin is mentioned. Proteins, fats, oils are mentioned as core material, preferably the core material of the microcapsule contains microcrystalline cellulose (MCC). The health-promoting substance contained in the capsules is probiotics. As probiotics are sensitive to environmental conditions, encapsulation is intended to ensure their stability by entrapping them in a coating. However, microcapsules according to this disclosure are not obtained from the fruit pulp. There is also no mention in this disclosure that the pectin mentioned comes from the fruit pulp.

It is known from the state of the art that emulsifiers such as Tween 80 and Span 80 (Polysorbate) are commonly used stabilizers for obtaining nanocapsules [15]. Tween (or polysorbate) is a non-ionic surfactant with a specific odour, slightly bitter. It is a partially esterified polyoxyethylene sorbitan fatty acid. It is widely used as an emulsifier and solubiliser especially for oils. It is generally considered to be a non-toxic ingredient, but its addition can affect the taste and aroma of the resulting product. One of the challenges in the field of the invention is the ability to manufacture products containing moderate amounts of unstable health-promoting oils while ensuring the stability and thus the properties of these oils.

The many years of scientific research carried out by the inventors of the present application have shown that natural biopolymers such as, for example, starch, chitosan, hyaluronic acid, alginate or pectin and their derivatives can successfully find application in the production of micro/nanoencapsulants. Patent PL242594 of the inventors of the present application discloses microcapsules in the form of a spherical or ellipsoidal solid, with an average diameter of 0.1 µm to 100 µm having a biodegradable membrane with a thickness of 5 nm to 100 nm, encapsulating a core, wherein the microcapsule membrane contains one or more biodegradable polysaccharides, namely: hyaluronic acid present in an amount of up to 3% by weight, preferably up to 2% by weight, especially from 0.5 to 1.5% by weight, or chitosan present in an amount up to 4% by weight, preferably up to 2% by weight, especially from 1.5 to 2% by weight, or alginate present in an amount up to 4% by weight, preferably up to 3% by weight, especially from 1 to 2.5% by weight, and the core is a cosmetic composition containing purified ozonated vegetable oil containing unsaturated fatty acids. The patent also discloses a film of biodegradable polysaccharides containing such microcapsules.

During their work, the research group was surprised to observe that pectins naturally found in fruit pulp enable the production of nano/microcapsules in such a way that the gelling pectin forms a coating that traps an emulsion of health-promoting ingredients. Thus, the present invention makes use of the pectin contained in the fruit pulp to obtain a coating of nano/microcapsules containing the biologically active ingredients in their core, whereby the pectin is not previously extracted from the fruit and supplied as an independent agent but is introduced into the method of obtaining the micro/nano-capsules together with the pulp. Surprisingly, it was found that in such cases micro/nanocapsules are formed. The remaining ingredients of the pulp form a matrix for the capsules and provide the flavor and aroma base of the product.

Therefore, the aim of the invention was to develop stable nano/microcapsules and a robust product containing them, using fruit pulp with health-promoting properties, ensuring the stability of the composition of natural bioactive substances trapped inside them. The invention also aims to develop a simple method for obtaining such nano/microcapsules and products containing them. A further aim of the invention is also to use such products for therapeutic purposes, e.g. for bacterial infections of the throat.

The subject matter of the invention are nano/microcapsules with an average diameter of 0.1 µm to 5 µm having a coating with a thickness of 5 nm to 100 nm encapsulating a core, characterised in that the nano/microcapsule coating contains pectin provided for the preparation of nano/microcapsules in the fruit pulp, while the core consists of a water-oil nanoemulsion containing bioactive components. Nano/microcapsules are in the form of a spherical or ellipsoidal solid with a liquid core.

Preferably, the bioactive ingredients are the components contained in natural oils, which constitute the oil phase of the nanoemulsion. Preferably, the oils are olive oil, sea buckthorn oil, pomegranate seed oil, apricot kernel oil, wild rose oil, hemp CBD oil, almond oil, cinnamon oil, cod liver oil, walnut oil, sesame oil, clove oil, or mixtures thereof.

Preferably, the nano/microcapsules contain additional bioactive ingredients, which are ingredients contained in bee products such as e.g. propolis, royal jelly, bee pollen, or mixtures thereof; ingredients contained in plant extracts and juices such as e.g. oregano extract, concentrated ashwagandha juice, concentrated pomace juice, burdock extract, hibiscus extract, turmeric extract, ginger extract, or mixtures thereof; oil- and/or water-soluble nanoemulsion vitamins; or mixtures thereof. The mixtures can be composed in almost any way, taking into account the taste and smell of the final product, wherein an oil and water component must be provided to create a nanoemulsion. The aqueous component can be pure water or an aqueous solution with bioactive substances.

Preferably, the bioactive ingredients are a mixture of propolis and sea buckthorn oil.

Preferably, the fruit is selected from a group including plums, currants, cherries, apples, cherries, apricots, raspberries, strawberries, chokeberries, peaches or mixtures of their flesh.

The subject matter of the invention is also a health-promoting product in solid or semi-liquid form characterised in that it contains nano/microcapsules as defined above embedded in a matrix constituting the fruit pulp. Preferably, the health-promoting product is in the form of films, pastilles, mousses, gels and chewing gums.

The subject matter of the invention is also the method of obtaining nano/microcapsules defined above comprising the following steps:
a. preparation of crushed fruits and their mechanical homogenization;
b. preparation of a nanoemulsion from a health-promoting oil and an aqueous solution containing bioactive ingredients using ultrasound homogenisation for 10 minutes at 10-15°C;
c. ultrasonic homogenization for 10 minutes at a temperature of 22°C of the fruit pulp obtained in stage a. with the nanoemulsion obtained in stage b. to form nano/microcapsules, wherein the weight ratio of fruit pulp to nanoemulsion is 6.25:1;
d. preparation of the mass to form the final product by mechanical homogenisation of the fruit pulp obtained in step a. with the nano/microcapsules formed in step c.

The invention solves several technical problems. Among others, there is the significant problem of the low stability of natural health-promoting oils. There is also the issue of having to use stabilisers/emulsifiers to stabilise such oils and it is well known that the fewer artificial additives the healthier the product. The technical problem that the invention solves is also the need for special gelling agents for the manufacture of nano/microcapsules. There is also the problem of the unpleasant taste and smell of some bioactive ingredients that are beneficial to health, which, in the case of the invention, can be easily encapsulated.

A food product with such nano/microcapsules could be an edible film that can be formed into any shape to suit the consumer. The resulting product can be shaped into pastilles or other almost arbitrary shapes to increase the product's marketability. One of the final products may also be a semi-liquid product, a mousse, which can be obtained without drying the fruit mass after the capsules have been produced. However, the product then contains a significant amount of water and should be sterilised and/or pasteurised.

The invention which provides, on the one hand, a tasty product of natural origin from fruit and, on the other hand, the use of the properties of pectin naturally present in fruit, has enabled the encapsulation of health-promoting ingredients such as vegetable oils and other substances known for their medicinal properties, such as bee products, plant extracts, or vitamins. The health-promoting nature of the product according to the invention is therefore twofold: from the fruit and the natural ingredients contained therein, and from the encapsulated additional natural bioactive ingredients. In addition, nano/microcapsules provide stability for the ingredients in the core, which manifests itself in the absence of the need for stabilisers commonly used in food products to stabilise health-promoting oils. Preliminary studies have shown that, despite encapsulating the bioactive ingredients, they continue to function as intended and that securing them in capsules increases their shelf life during storage.

The phrase nano/microcapsules should be understood as a mixture of nanometric and micrometric sized capsules. Nano/microcapsules take the form of a spherical or ellipsoidal solid with a liquid core, with an average diameter ranging from 0.1 µm to 5 µm. Both nanocapsules and microcapsules fall within this diameter size range. In general, these products can also be understood as capsules of a certain size of average diameter. Depending on the ingredients used, the proportion of nanocapsules and microcapsules can vary in the mixture. Spherical form should be understood as spherical or as close to spherical. The same applies to the ellipsoidal shape - this term refers to any possible shape of an elongated, irregular sphere, including those with a corrugated surface.

The structure of the nano/microcapsules according to the invention is as follows. They have a pectin coating and a core containing biologically active ingredients contained in a water-oil nanoemulsion. The biologically active ingredients are derived from:
- natural vegetable oils, e.g. selected from the following group: sea buckthorn, walnut, flax, almond, cinnamon, wild rose, sesame, CBD, cinnamon, clove, fish oil - an ingredient necessary to achieve emulsion;
- additional ingredients such as:
   i. oil-soluble bee products such as propolis, royal jelly and bee pollen
   ii. oil-soluble vitamins such as A, D3, K, E, B-group
   iii. water-soluble substances and extracts, e.g. selected from the group: folic acid, concentrated fruit juices, concentrated pomace juices, ashwagandha juice concentrate, burdock extract, hibiscus extract, curcumin extract, ginger extract; water-soluble vitamins.

Any mixture of bioactive ingredients can be used. The main limitation in composing the core of micro/nanocapsules according to the invention is the final taste, smell of the product, and its intended use.

Of course, the above list of compounds should not be considered as limiting the scope of protection. Other bioactive ingredients that dissolve in oil or water can also be introduced into the core of the nano/microcapsules. To ensure the versatility of the capsule system claimed here, the core contains an oil-in-water emulsion to ensure that oil-soluble and/or water-soluble components can be dissolved in the core. The basic version of the nano/microcapsules contains only health-promoting oil/oils in the form of an oil-in-water emulsion.

The use of ultrasound to emulsify oil in water allows a stable emulsion to be obtained by breaking down oil droplets to sizes ranging from several dozen to several hundred nanometers, and the fruit pulp containing polysaccharide - pectin - surrounds the nanoemulsion droplets, "trapping" the emulsified components and enabling the production of stable nano/microcapsules according to the invention.

To produce edible films, pastilles or other forms of products containing nano/microcapsules, fruits such as plums, currants, cherries, apples, dogwood, apricots, raspberries, strawberries, chokeberries, peaches can be used, individually or mixed in various proportions depending on the desired organoleptic and physicochemical properties. They can also be pectin-containing fruits other than those listed here. It is noteworthy that only fruit pulp is used to produce nano/microcapsules according to the invention enriched (in the core) with biologically active and health-promoting ingredients of natural origin. It provides not only flavour and texture but also pectin, which acts as a gelling polysaccharide that forms the nano/microcapsule coating. It not only provides taste and texture, but also pectin, which acts as a gelling polysaccharide forming nano/microcapsule coatings. In addition, there are flavourings and fragrances, as well as health-promoting agents to ensure the product's favourable composition and texture.

In addition, it was found that the inventive encapsulation technique used makes it possible to obtain stable nano/microcapsules without the addition of commonly used synthetic stabilisers used during encapsulation processes (emulsifiers, stabilisers). At the same time, a favourable taste, texture and positive association with fruit is ensured.

The subject matter of the invention is illustrated by the following examples of implementation and the drawing, which individual figures show:
Fig. 1 schematically depicts a cross-section of a section of the product in the form of a film according to the invention;
Fig. 2 schematically depicts the microcapsule according to the invention with the interior visualised;
Fig. 3 shows a microscopic image of a film containing nano/microcapsules according to the invention;
Fig. 4 shows the films and pastilles obtained;
Fig. 5 shows the effect of the film according to product 1 on *S. aureus.*

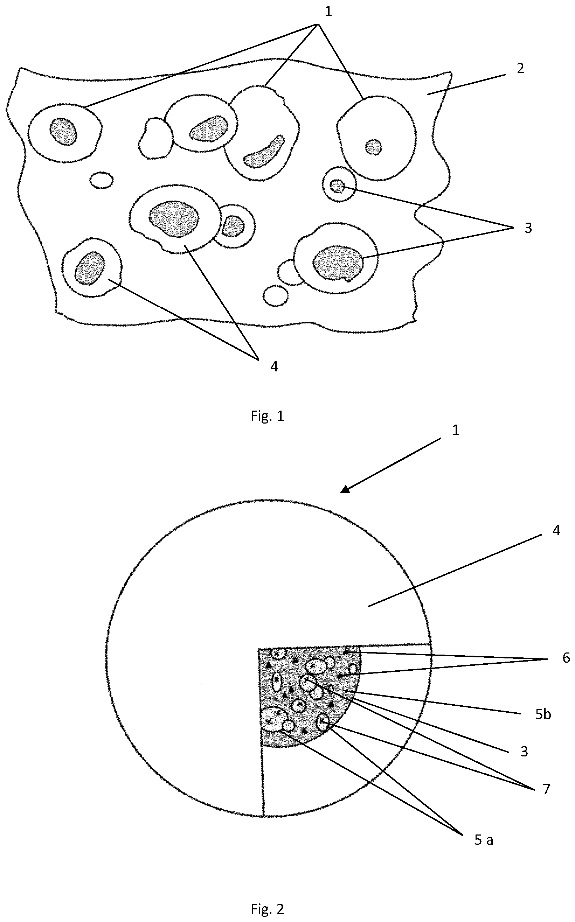

Fig. 2 shows microcapsule 1 as a spherical solid, with an average diameter of 0.2 µm having a 25 nm thick coating. Capsule 1 is composed of a pectin coating 4 covering a liquid core 3. Core 3 is a water-oil nanoemulsion. The oil nanoparticles labelled 5a are dispersed in aqueous solution 5b. Both nanoemulsion phases can contain bioactive components 6, 7 which depends on whether they are oilor water-soluble. Nanocapsules are, of course, analogous in structure but smaller in size than microcapsules. The nano/microcapsules are contained in matrix 2, which is fruit pulp. Nano/microcapsules in the matrix are shown in Fig. 1.

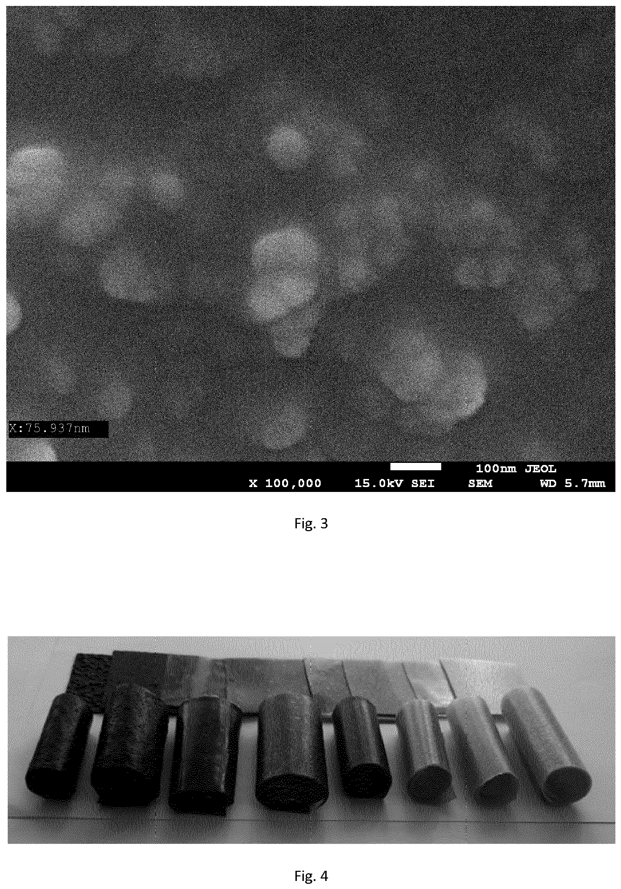

Fig. 4 shows the form of the final health-promoting product according to the invention. The figure shows the films (back) and the product formed into pastilles (front).

Advantages of the invention:
- only natural ingredients were used to prepare the products according to the invention,
- fruit pulp is used to make the products according to the invention, which provides a taste, aroma and texture positively associated with fruit, as well as providing the natural nutrients and health elements contained in fruit,
- gelling properties of pectins contained in fruit were used to obtain nano/microcapsules; there is no need to use other ingredients, such as previously isolated pectins,
- nano/microcapsules as well as the oils they contain are stable/robust without the addition of artificial stabilisers/emulsifiers,
- nanoemulsion contained in the core of the capsules can contain both water-soluble and oil-soluble ingredients,
- the possibility of encapsulating many types of bioactive substances,
- every fruit contains pectin, which gives enormous scope for the composition, flavour, aroma and texture of the final products, depending on the characteristics of the specific fruit,
- encapsulation of bioactive substances ensures their robustability and stability over time,
- bioactive ingredients are released from the capsules over a long section of the digestive tract, starting from the mouth and ending in the intestine,
- the release of bioactive ingredients over a long section of the digestive tract ensures, for example, antibacterial action in the throat and allows them to pass into the bloodstream from the small intestine (gelatinized pectin in the coating can reach the intestines),
- the final product is stable over time,
- depending on the ingredients, the product may have bactericidal or other effects,
- encapsulating active substances with an unpleasant taste or smell diminishes this taste and smell, making it possible to produce products for children or people sensitive to tastes/odours,
- incidentally, the product provides pectin, i.e. fiber - an essential ingredient for the proper functioning of the body,
- functional foods can be designed for different consumer groups, e.g. for children (masking unpleasant tastes and smells), for pregnant women (folic acid), for the treatment of conditions such as sore throat, or to supply the bloodstream with health-promoting ingredients,
- the stability of the bioactive ingredients means that smaller quantities can be used with the same beneficial effect,
- the products can be in different forms, the film can be formed into different shapes, they can be pastilles, lollipops, soluble chewing gum, mousses (undried film), etc.,
- easy method of producing nanoemulsions, nano/microcapsules and the final product, simple equipment, no need for additional chemicals/food additives,
- the use of indigenous, local and seasonal fruit.

### Examples

The following equipment and reagents were used in the examples.
GÖTZE & JENSEN TB901X cooking blender - https://www.mediaexpert.pl/agd-male/do-kuchni/blendery-kielichowe/blender-gotujacy-gotze-jensen-tb901x
Mechanical homogeniser - Polytron PT 2500 E, Kinematica AG, Malters, Switzerland
Ultrasonic homogeniser - 20 kHz, Sonopuls HD 4200, Bandelin, Berlin, Germany
Dryer Dehydrator Zyle Professional Medium 700W - https://sklep.ronic.pl/dehydratory/1302-dehydrator-zyle-professional-medium-suszarka-do-owocow-i-warzyw-4779025998710.html Electron microscope - JEOL 7550 scanning electron microscope (Akishima, Tokyo, Japan) Micrometer - catalogue no. 805.1301 (Sylvac SA, Crissier, Switzerland), with a resolution of 0.001 mm Maldi-TOF (Matrix-assisted laser desorption/ionisation time-of-flight) - Bruker, Billerica, MA, USA https://. r.com/en/products-and-solutions/mass-spectrometry/maldi-tof.html
Propolis https://allegro.pl/oferta/ekstrakt-propolisowy-bezalkoholowy-40-100ml-13199378011?bundle_id=48389c7e-efa3-40b0-88d6-277d537a6b16
Sea-buckthorn oil 100% sea buckthorn oil, 100 ml - FutuNatura.co.uk

### Example 1. Preparation of the fruit pulp

500 g of apples (Lubo variety, country of origin: Poland) and 1000 g of plums (domestic plum, Prezydent variety, country of origin: Poland) were used to make the fruit pastilles. The apples were peeled and cored, and the plums were pitted, leaving the skin on. The fruits, each kind individually, were crushed with a cooking blender at 25 °C for 15 minutes then subjected to mechanical homogenisation for 5 minutes at 25 °C.

### Example 2. Preparation of nanoemulsions

A nanoemulsion of bioactive ingredients was prepared independently. For this purpose, 20 g of a mixture of propolis (40% water extract) and sea buckthorn oil (in a 1:1 weight ratio, cooled in an ice bath) was subjected to ultrasonic homogenization for 10 minutes at a temperature of 10-15 °C.

### Example 3. Preparation of plum foil

To 50 g of homogenized plum mass obtained as in Example 1, 8 g of nanoemulsion obtained according to Example 2 was added, and the mass was subjected to ultrasonic homogenization for 10 minutes at room temperature (approx. 22 °C) to form and stabilize nano/microcapsules. The resulting mixture was combined using a mechanical homogeniser for 15 minutes at room temperature (approx. 22 °C) with 550 g of homogenised plum pulp, poured into a tray, and dried for 24 h at 35 °C. The resulting film was labelled as **product 1.** Observation of the product showed that there were no visible droplets/layers of oil separating from the flesh, indicating that the oil was bound into nano/microcapsules.

### Example 4. Preparation of the film according to the invention from a mixture of apples and plums

To 50 g of homogenized plum mass obtained as in Example 1, 8 g of nanoemulsion obtained as in Example 2 was added, and the mass was subjected to ultrasonic homogenization for 15 min at room temperature (approx. 22 °C) to stabilize and form capsules. The resulting mixture was combined (mechanical homogeniser) with 850 g of homogenised mass (500 g apples + 350 g plums), poured into a tray, and dried for 24 h at 35 °C. The resulting film was labelled as **product 2.** Observation of the product showed that there were no visible droplets/layers of oil separating from the flesh, indicating that the oil was bound into nano/microcapsules.

### Example 5. Obtaining control samples

The homogenised plum fruit was spread onto a 200 g tray and dried for 24 h at 35 °C. The film thus obtained is **control sample 1.**

The homogenised plum fruit was combined with apple in a 1:1 ratio using a mechanical homogeniser. The mixture was poured into a 200 g tray and dried for 24 h at 35 °C. The film thus obtained is **control**

### sample 2.

An attempt was made to obtain control samples as mixtures of:
- fruit pulp with vegetable oils and
- fruit pulp with a blend of vegetable oils and additional active substances, as well as
- fruit pulp with various nanoemulsions but without ultrasonic homogenization - a control sample prepared in the same way as for product 1 was named control sample 3.

Such mixtures were poured into a 200 g tray and subjected to drying for 24 h at 35 °C. However, a number of final products made in this way were not suitable for further testing. The pulp and oil phase separated, with oil appearing on top of the pulp, increasing over time.

### Example 6. Microscopic images

In order to determine whether nano/microcapsules of defined size and morphology were obtained, microscopic images of product 1 were taken. Samples were analysed for nanoparticle size and morphology using a JEOL 7550 scanning electron microscope (Akishima, Tokyo, Japan). Prior to measurements, the sample was coated with a 20 nm layer of chromium (Cr) using a K575X Turbo Sputter Coater (Emitech, Ltd., Kent, UK) to increase the conductivity of the sample.

The nano/microcapsules were found to have an average diameter of 0.1 µm to 5 µm and an coating thickness of 5 nm to 100 nm.

Fig. 3 shows an electron microscope image for product 1 containing nano/microcapsules. This proves that microcapsules of the assumed form have been formed. In addition, microscopic images showed the presence of nanometric oil particles in the emulsion found in the core of the capsules (e.g. as encountered in a broken capsule).

An analogous image was observed for product 2.

### Example 7. Mechanical properties, thickness and mechanical properties of the film

Products 1 and 2 according to the invention as well as control samples 1 and 2 were tested for mechanical properties (strength, extensibility) in order to demonstrate whether the presence of capsules affects the organoleptic characteristics of the obtained products.

The thickness of the rigid flat samples (films) was measured with a micrometer to the nearest 0.025 mm (0.001 inch) at several points. The results of the measurements were used to calculate the breaking strength of the sample.

Samples for textural analysis were prepared according to the standards [16]. The mechanical properties of the film were determined using a TA-XT plus texture analyser (Stable Micro Systems, Haslemere, UK). Film strips (35 × 6 mm2) were cut out and then placed in the holders of the measuring instrument. The initial distance between the holders was 20 mm and the stretching rate was 2 mm/min. Tensile strength (TS) was calculated by dividing the maximum force at film break by the crosssectional area of the film. The elongation percentage (EAB) was calculated by dividing the elongation at break by the initial distance between the handles and multiplying by 100 [17].

**Table 1. Thickness and mechanical properties of products according to the invention and control samples.**

| Sample | Thickness (mm) | TS (MPa) | EAB (%) |
|---|---|---|---|
| Control 1 | 0.526 ± 0,022 | 0.71 ± 0,15 | 14.38 ± 2,78 |
| Control 2 | 0.452 ± 0,038 | 0.75 ± 0,39 | 10.33 ± 2,11 |
| Product 1 | 0.554 ± 0,028 | 0.64 ± 0,19 | 3.65 ± 1,37 |
| Product 2 | 0.545 ± 0,026 | 0.51 ± 0,15 | 12.13 ± 4,42 |

| | | | |
|---|---|---|---|
| TS-tensile strength, EAB-film elongation. | | | |

The thickness of the obtained products was similar. This parameter can be modified according to need and purpose. Larger, thicker products can be created for adults and smaller products for children. However, it is worth noting that the thickness will determine the hardness and elongation of the product.

The control samples had a higher hardness, tensile strength (TS) which suggests that they will be more difficult to bite, chew or cut than the products according to the invention. The influence of the additives and the presence of micro/nanocapsules is therefore beneficial as it contributes to a reduction in the stiffness of the samples, which results in better chewability of the product in the mouth. The EAB parameter describes the flexibility of the sample. A lower value of the EAB parameter was observed for product 1 according to the invention relative to control sample 1. Lower elasticity means less stretchability. The samples are more compact and will change shape to a lesser extent when the sample is sucked on the tongue. Product 2 shows a higher EAB than control 2. This product is looser than the control, it will change shape when chewed in the mouth. The experiments described here demonstrate the possibility of influencing the consistency of a product by its composition, which provides the possibility of adapting it to consumer preferences.

### Example 8. Antimicrobial testing

Products 1 and 2 according to the invention and control samples 1 and 2 were subjected to microbiological tests to demonstrate their antimicrobial activity. The expected bactericidal effect is due to the fact that the components in their composition contain a number of substances with bactericidal properties. Propolis contains polyphenols (phenolic acids, flavonoids) and terpenoids, as well as aromatic acids (e.g. benzoic, salicylic, p-coumaric). They have antimicrobial effects by increasing the permeability of bacterial cell membranes, decreasing ATP production, causing disruption of membrane potential, reducing motility, and stimulating the immune response. Sea buckthorn oil, in turn, contains vitamin C in a concentration of approximately 1 to 5 mg/g, phenolic substances (including phenolic glycosides, flavonoids, phenolic acids), glucosinolates, isothiocyanates, carotenoids, and vitamin E. These substances have similar effects to those mentioned above.

The test described here aims to demonstrate that the fruit products obtained, with the composition presented, have bactericidal potential, i.e., through contact with bacterial cells, they inhibit their growth.

The antimicrobial efficacy test was performed using seven microbial isolates from the upper respiratory tract (i.e., throat, tonsils, nasopharynx) of children.

Bacterial isolates were obtained by swabbing the throat, tonsils, and nasopharynx with a sterile swab. After collecting the swab, the material was sown reductively on selective media, i.e.: Columbia Blood Agar, Baird-Parker Agar, UTI Agar, CNA Agar. After an incubation period of 24 hours at 36 °C, characteristic colonies were isolated for the bacteria listed in the table below. Gram staining was performed, microscopic observations were made, and after selecting the colonies, species identification was carried out using MALDI-TOF spectrometry [18, 19].

The reference strains were from the ATCC (American Type Culture Collection) or NCTC (National Collection of Type Cultures, UK) collections.

The microbial species tested included:
*Staphylococcus aureus* (5 strains including one reference strain). It is estimated that about 40% of the population are permanently or periodically asymptomatic carriers of S. aureus (nose, skin, throat). Carriage may be a starting point for infection in situations of reduced immunity. It most commonly causes skin and soft tissue infections, gastrointestinal infections and systemic infections (e.g. respiratory tract, myocardium, staphylococcal septicaemia). It has numerous virulence factors: enterotoxins, hemolysins, coagulase, fibrinolysin penicillinase, CF factor, leukocidin. Antibiotic resistance is common: beta-lactamase production, methicillin resistance, vancomycin and aminoglycoside resistance.
*Acinetobacter radioresistens* (1 strain). Gram-negative bacterium isolated from a patient with throat symptoms
*Enterobacter* spp. (1 strain). Bacteria responsible for hospital infections and, less frequently, nonhospital infections, including respiratory and other infections.

**Table 2. Origin of tested strains**

| | |
|---|---|
| *S. aureus* 1 | *origin - throat, year of birth 2014* |
| *S. aureus* 2 | *origin - nasopharynx, year of birth 2016* |
| *S. aureus* 3 | *origin - throat, year of birth 2017* |
| *S. aureus* 4 | *origin - throat, year of birth 2017* |
| *S. aureus* ATCC reference | *ATCC* 25923 *https:*//*www.atcc.org*/*products*/*25923* |
| *A. radioresistens* | *origin - throat, year of birth 2016* |
| *Enterobacter* spp. | *origin - oral cavity*/*throat, year of birth 2016* |

The strains tested, which were not reference strains, were obtained from different individuals by isolating bacteria using the technique described above.

The study was carried out based on the guidelines used for testing antibiotic resistance of microorganisms, issued by the National Reference Centre for Antimicrobial Drug Resistance (NRLC). Standard suspensions of the test microorganisms were prepared, with a MacFarland density of 0.5. From the suspensions, a surface, catchment culture was performed on Mueller-Hinton II medium. The test films, cut into 5mm squares, were then placed on the surface of the media with the prepared cultures. The films were previously sterilised under a UV lamp (sterilisation time - min. 30 minutes). The dishes were incubated, first overnight at room temperature to allow the active substance to be released into the medium, and then overnight at 36°C. After the incubation time, zones of growth inhibition were observed around the foil. The diameter of the growth inhibition zones was measured and the results are given in mm.

**Table 3. Size of growth inhibition zones under the influence of pastilles (mm).**

| | **Control 1** | **Product 1** | **Control 2** | **Product 2** |
|---|---|---|---|---|
| *S. aureus* 1 | 20 | 42 | nd | nd |
| *S. aureus* 2 | 20 | 25 | 10 | 12 |
| *S. aureus* 3 | 16 | 20 | 10 | 12 |
| *S. aureus* 4 | 12 | 13 | 12 | 12 |
| *S. aureus* ATCC reference | 32 | 36 | 15 | 20 |
| *A. radioresistens* | 15 | 17 | 15 | 15 |
| *Enterobacter* spp. | 15 | 17 | nd | nd |

| | | | | |
|---|---|---|---|---|
| nd - not tested | | | | |

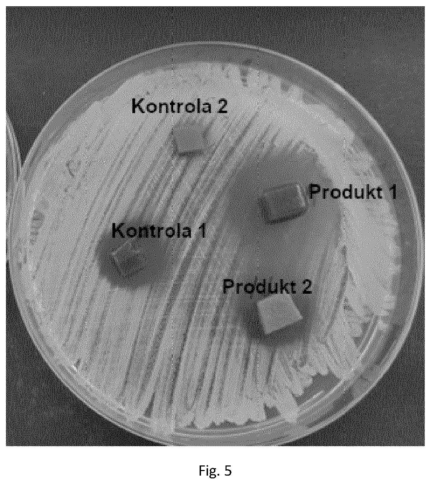

The effect of film by product 1 on *S. aureus* 1 is shown in Fig. 5 where zones of inhibited reproduction of these bacteria are observed. In Fig. 5 it can be seen that there is a larger zone of inhibition around product 1 than around control 1. In Fig. 5 it can also be seen that there is a larger zone of inhibition around product 2 than around control 2.

The products show inhibition of the growth of many pathogenic bacteria that cause throat infections. This demonstrates that the bioactive substances released from the capsules in the mouth during chewing will have a health-promoting effect on sore throats.

### Example 9. Product 1 stability test

Stability testing of product 1 was performed by leaving it on the shelf at room temperature for six months. After three months and six months, the antioxidant properties were tested according to a known procedure.

### Preparation of samples for analysis

Methanolic extracts of the test samples were prepared for analysis of total polyphenol content and antioxidant properties. The extraction process was carried out using an ultrasonic bath (Polsonic, Warsaw, Poland) (30 minutes at 25 °C). Samples were treated with 96% methanol. The resulting supernatants (methanol extracts) were used for further analysis.

### Antioxidant activity was determined using ABTS+, DPPH, and FRAP assays.

According to the methodology proposed by Re et al. [20], analysis of antioxidant activity was carried out using the cationic radical ABTS+ (2,2'-azinobis-3-ethylbenzothiazoline-6-sulfonic acid). The reaction mixture consisted of the addition of the sample (0.03 ml) and the ABTS radical solution to water (3.0 ml). Absorbance at 734 nm against distilled water was measured after 6 minutes of reaction.

The scavenging activity was measured by the elimination of DPPH (1,1-diphenyl-2-picrylhydrazyl) free radicals [21]. The reaction mixture consisted of the sample (0.5 ml) and a solution of the DPPH radical in methanol (2.0 ml). Absorbance at 517 nm against methanol was measured after 10 minutes of reaction.

The analysis of antioxidant capacity by FRAP was based on the methodology proposed by Benzi and Strain [22]. To the samples to be analysed, 3 ml of 0.5 ml FRAP reagent solution was added and mixed. The absorbance of the solutions was measured after 10 minutes at 593 nm against distilled water. Antioxidant activity as determined by ABTS+, DPPH and FRAP methods was expressed in mmol TE/100 g (Trolox equivalent - an α-tocopherol analogue).

### Total polyphenol content

The total polyphenol content of the samples obtained was assessed using the Folin-Ciocalteu phenol reagent by the method described by Singleton et al. [23]. The reaction mixture contained extract (0.1 ml), Folin-Ciocalteu reagent (0.2 ml), distilled water (2.0 ml) and 20.0% (w/v) sodium carbonate solution (1.0 ml). The absorbance of the samples was measured at 765 nm against distilled water. Total polyphenol content (TPC) was expressed as gallic acid equivalent (GAE) in milligrams per 100 g of samples.

ABTS, DPPH, FRAP and TPC were measured in triplicate using a spectrophotometer (Nicolet Evolution 300, Thermo, Waltham, MA, USA).

Similarly, control sample 3 was tested.

Preliminary studies have shown that product 1 shows undiminished antioxidant properties over the time studied. Furthermore, organoleptic evaluation leads to the conclusion that the condition of the product has not deteriorated, it still has a good appearance, colour, taste, fruit aroma, consistency, and no oil spots are visible.

In contrast, control sample 3 shows a loss of antioxidant properties by 10% after three months and up to 30% after six months. Furthermore, organoleptic assessment leads to the conclusion that the condition of the product deteriorates over time, with a rancid oil odour emanating from the flesh in the form of exudates/oil droplets. The product did not have a good appearance from the start, with the appearance, taste, smell and texture deteriorating over time.

Tests show that active ingredients (oils and additives dissolved in oil or the free phase of a nanoemulsion) are contained in nano/microcapsules, which ensures product stability during storage. At the same time, the product is edible and when chewed, the capsule coating breaks down and the active substances are partially released into the mouth, where they can exert their antibacterial effect. They are then digested by gastric juice and enzymes, ensuring a slow release of the ingredients. There is no risk of substances not being released from the capsules.

### Example 10. Other products

A number of other films or pastilles were prepared in accordance with the procedure described for products 1 and 2, containing the following ingredients:
- apple and plum, vitamin D as dissolved component in sea buckthorn oil + oregano extract
- apple and plum, cinnamon oil alone
- apple, water-soluble folic acid + sea buckthorn oil
- pear and plum, CBD oil + sea buckthorn oil
- currant and apple, concentrated water-soluble pomace juice + rosehip oil
- peach and apple, ashwagandha juice concentrate + almond kernel oil
- plum and peach, water-soluble burdock extract + walnut oil
- plum and peach, hibiscus extract + sea buckthorn oil
- apple and plum, water-soluble blend of curcumin and ginger extract + rosehip oil
- apple and plum, sesame oil alone
- apple and currant, linseed oil alone
- apple, cinnamon oil alone
- plum and peach, clove oil alone
- apple and plum, cod liver oil

All products have been pre-tested for antibacterial properties, stability, mechanical properties. They showed analogous characteristics to products 1 and 2 (not shown here).

### Example 11. Preparation of the plum mousse

To 50 g of homogenized plum mass obtained as in Example 1, 8 g of nanoemulsion obtained according to Example 2 was added, and the mass was subjected to ultrasonic homogenization for 10 minutes at room temperature (approx. 22 °C) to form and stabilize nano/microcapsules. The mixture was combined using a mechanical homogenizer for 15 minutes at room temperature (approx. 22 °C) with 550 g of homogenized plum pulp, poured onto a tray, and dried for 4 hours at 35 °C. A loose-textured mousse was obtained. The product was pasteurized for 10 minutes in an autoclave (CHE-32 electrouniversal food autoclave) at 90 °C. Observation of the product showed no visible droplets/layers of oil separating from the pulp, which indicates that it is bound in nano/microcapsules.

Bibliography:
1. Negrean, O.R.; Farcas, A.C.; Pop, O.L.; Socaci, S.A. Blackthorn-A Valuable Source of Phenolic Antioxidants with Potential Health Benefits. Mol. 2023, Vol. 28, Page 3456 2023, 28, 3456, doi:10.3390/MOLECULES28083456.
2. Haris, S.; Alam, M.; Galiwango, E.; Mohamed, M.M.; Kamal-Eldin, A.; Al-Marzouqi, A.H. Characterization Analysis of Date Fruit Pomace: An Underutilized Waste Bioresource Rich in Dietary Fiber and Phenolic Antioxidants. Waste Manag. 2023, 163, 34-42, doi:10.1016/J.WASMAN.2023.03.027.
3. Kaur, C.; Kapoor, H.C. Antioxidants in Fruits and Vegetables - the Millennium's Health. Int. J. Food Sci. Technol. 2001, 36, 703-725, doi:10.1111/J.1365-2621.2001.00513.X.
4. Rahaman, M.M.; Hossain, R.; Herrera-Bravo, J.; Islam, M.T.; Atolani, O.; Adeyemi, O.S.; Owolodun, O.A.; Kambizi, L.; Da 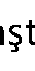 tan, S.D.; Calina, D.; et al. Natural Antioxidants from Some Fruits, Seeds, Foods, Natural Products, and Associated Health Benefits: An Update. *Food Sci. Nutr.* **2023,** *11*, 1657-1670, doi:10.1002/FSN3.3217.
5. Jideani, A.I.O.; Silungwe, H.; Takalani, T.; Omolola, A.O.; Udeh, H.O.; Anyasi, T.A. Antioxidant-Rich Natural Fruit and Vegetable Products and Human Health. Int. J. Food Prop. 2021, 24, 41-67, doi:10.1080/10942912.2020.1866597.
6. He, J.; Yang, S.; Goksen, G.; Cong, X.; Rizwan Khan, M.; Zhang, W. Functionalized Pectin/Alginate Food Packaging Films Based on Metal-Phenol Networks. Food Biosci. 2024, 58, 103635, doi:10.1016/J.FBIO.2024.103635.
7. El-Saadony, M.T.; Zabermawi, N.M.; Zabermawi, N.M.; Burollus, M.A.; Shafi, M.E.; Alagawany, M.; Yehia, N.; Askar, A.M.; Alsafy, S.A.; Noreldin, A.E.; et al. Nutritional Aspects and Health Benefits of Bioactive Plant Compounds against Infectious Diseases: A Review. Food Rev. Int. 2023, 39, 2138-2160, doi:10.1080/87559129.2021.1944183.
8. Samtiya, M.; Aluko, R.E.; Dhewa, T.; Moreno-Rojas, J.M. Potential Health Benefits of Plant Food-Derived Bioactive Components: An Overview. Foods 2021, Vol. 10, Page 839 2021, 10, 839, doi:10.3390/FOODS10040839.
9. Ud Din, S.R.; Saeed, S.; Khan, S.U.; Kiani, F.A.; Alsuhaibani, A.M.; Zhong, M. Bioactive Compounds (BACs): A Novel Approach to Treat and Prevent Cardiovascular Diseases. Curr. Probl. Cardiol. 2023, 48, 101664, doi:10.1016/J.CPCARDIOL.2023.101664.
10. Kaparapu, J.; Pragada, P.M.; Geddada, M.N.R. Fruits and Vegetables and Its Nutritional Benefits. Funct. Foods Nutraceuticals 2020, 241-260, doi:10.1007/978-3-030-42319-3_14.
11. Hanula, M.; Szpicer, A.; Górska-Horczyczak, E.; Khachatryan, G.; Pogorzelska-Nowicka, E.; Poltorak, A. Quality of Beef Burgers Formulated with Fat Substitute in a Form of Freeze-Dried Hydrogel Enriched with AçAi Oil. Mol. 2022, Vol. 27, Page 3700 2022, 27, 3700, doi:10.3390/MOLECULES27123700.
12. Hanula, M.; Szpicer, A.; Górska-Horczyczak, E.; Khachatryan, G.; Pogorzelski, G.; Pogorzelska-Nowicka, E.; Poltorak, A. Hydrogel Emulsion with Encapsulated Safflower Oil Enriched with Açai Extract as a Novel Fat Substitute in Beef Burgers Subjected to Storage in Cold Conditions. Mol. 2022, Vol. 27, Page 2397 2022, 27, 2397, doi:10.3390/MOLECULES27082397.
13. Turek, K.; Khachatryan, G.; Khachatryan, K.; Krystyjan, M. An Innovative Method for the Production of Yoghurt Fortified with Walnut Oil Nanocapsules and Characteristics of Functional Properties in Relation to Conventional Yoghurts. 2023**.**
14. Janik, M.; Hanula, M.; Khachatryan, K.; Khachatryan, G. Nano-/Microcapsules, Liposomes, and Micelles in Polysaccharide Carriers: Applications in Food Technology. Appl. Sci. 2023, Vol. 13, Page 11610 2023, 13, 11610, doi:10.3390/APP132111610.
15. Acácio, B.R.; Prada, A.L.; Neto, S.F.; Gomes, G.B.; Perdomo, R.T.; Nazario, C.E.D.; Neto, E.S.; Martines, M.A.U.; de Almeida, D.A.T.; Gasparotto Junior, A.; et al. Cytotoxicity, Anti-Inflammatory Effect, and Acute Oral Toxicity of a Novel Attalea Phalerata Kernel Oil-Loaded Nanocapsules. Biomed. Pharmacother. 2024, 174, 116308, doi:10.1016/J.BIOPHA.2024.116308.
16. Janik, M.; Khachatryan, K.; Khachatryan, G.; Krystyjan, M.; Zarska, S.; Ciesielski, W. Preparation and Characterisation of Acid-Base-Change-Sensitive Binary Biopolymer Films with Olive Oil and Ozonated Olive Oil Nano/Microcapsules and Added Hibiscus Extract. Int. J. Mol. Sci. 2023, Vol. 24, Page 11502 2023, 24, 11502, doi:10.3390/IJMS241411502.
17. Krystyjan, M.; Khachatryan, G.; Ciesielski, W.; Buksa, K.; Sikora, M. Preparation and Characteristics of Mechanical and Functional Properties of Starch/Plantago Psyllium Seeds Mucilage Films. Starch/Staerke 2017, 69, 1700014, doi:10.1002/star.201700014.18. Lenart-Boroń A, Stankiewicz K, Czernecka N, Ratajewicz A, Bulanda K, Heliasz M, Sosińńska D, Dworak K, Ciesielska D, Siemińska I, et al. Wounds of Companion Animals as a Habitat of Antibiotic-Resistant Bacteria That Are Potentially Harmful to Humans-Phenotypic, Proteomic and Molecular Detection. International Journal of Molecular Sciences. 2024; 25(6):3121. https://doi.org/10.3390/ijms25063121
19. Lenart-Boroń A, Stankiewicz K, Dworak K, Bulanda K, Czernecka N, Ratajewicz A, Khachatryan K, Khachatryan G. Hyaluron-Based Bionanocomposites of Silver Nanoparticles with Graphene Oxide as Effective Growth Inhibitors of Wound-Derived Bacteria. International Journal of Molecular Sciences. 2024; 25(13):6854. https://doi.org/10.3390/ijms25136854
20. Re, R.; Pellegrini, N.; Proteggente, A.; Pannala, A.; Yang, M.; Rice-Evans, C. Antioxidant activity applying an improved ABTS radical cation decolorization assay. Free. Radic. Bio: Med. 1999, 26, 1231-1237.
21. Yen, G.C.; Chen, H. Antioxidant activity of various tea extracts in relation to their antimutagenicity. J. Agric. Food Chem. 1995, 43, 27-32.
22. Benzie I.F.F i Strain J.J. 1996. The Ferric Reducing Ability of Plasma (FRAP) as a Measure of "Antioxidant Power": The FRAP Assay. Analytical biochemistry 239, 70-76
23. Singleton, V.L.; Orthofer, R.; Lamuela-Raventós, R.M. Analysis of Total Phenols and Other Oxidation Substrates and Antioxidants by Means of Folin-Ciocalteu Reagent. In Methods in Enzymology; Elsevier: Amsterdam, The Netherlands, 1999; Volume 299, pp. 152-178.

## Claims

1. Nano/microcapsules with an average diameter of 0.1 µm to 5 µm having a coating with a thickness of 5 nm to 100 nm encapsulating a core, **characterised in that** the coating (4) of the nano/microcapsules (1) contains pectin provided for the preparation of the nano/microcapsules (1) in the fruit pulp, while the core (3) consists of a water-oil nanoemulsion containing bioactive components (6, 7).

2. Nano/microcapsules according to claim 1 **characterised in that** the bioactive ingredients are those contained in the natural oils, which constitute the oil phase of the nanoemulsion.

3. Nano/microcapsules according to claim 1 or 2 **characterised in that** the oils used are olive oil, sea buckthorn oil, pomegranate seed oil, apricot kernel oil, wild rose oil, CBD hemp oil, almond oil, cinnamon oil, cod liver oil, walnut oil, sesame oil, clove oil, or blends thereof.

4. Nano/microcapsules according to claim 1, 2 or 3 **characterised in that** they contain additional bioactive components, which are ingredients contained in bee products such as propolis, royal jelly, bee pollen or mixtures thereof; ingredients contained in plant extracts and juices such as, for example oregano extract, concentrated ashwagandha juice, concentrated pomace juice, burdock extract, hibiscus extract, turmeric extract, ginger extract, or mixtures thereof; oiland/or water-soluble nanoemulsion vitamins; or mixtures thereof.

5. Nano/microcapsules according to any of the above claims, **characterised in that** the bioactive ingredients are a mixture of propolis and sea buckthorn oil.

6. Nano/microcapsules according to any of the above claims, **characterised in that** the fruits are selected from the group including plums, currants, cherries, apples, cherries, apricots, raspberries, strawberries, chokeberries, peaches or mixtures of their flesh.

7. A health-promoting product in solid or semi-liquid form, **characterised in that** it contains nano/microcapsules (1) as defined in claims 1-6 embedded in a matrix (2) constituting the fruit pulp.

8. Pro-health product according to claim 7 **characterised in that** it is in a form of films, pastilles, mousses, gels, chewing gums.

9. A method of obtaining nano/microcapsules as defined in claims 1-6 comprising the following steps:
a. preparation of crushed fruits and their mechanical homogenization;
b. preparation of a nanoemulsion from a health-promoting oil and an aqueous solution containing bioactive ingredients using ultrasound homogenisation for 10 minutes at 10-15°C;
c. ultrasonic homogenization for 10 minutes at a temperature of 22°C of the fruit pulp obtained in stage a. with the nanoemulsion obtained in stage b. to form nano/microcapsules, where the weight ratio of fruit pulp to nanoemulsion is 6.25:1;
d. preparation of the mass to form the final product by mechanical homogenisation of the fruit pulp obtained in step a. with the nano/microcapsules formed in step c.
